(19) Europäisches Patentamt
European Patent Office
Office européen des brevets

(11) Publication number: **0 136 381**
**B1**

(12) **EUROPEAN PATENT SPECIFICATION**

(45) Date of publication of patent specification: **08.06.88**

(51) Int. Cl.$^4$: **C 08 L 23/02,** C 08 K 5/06 // (C08L23/02, C08K5:06)

(21) Application number: **83306017.1**

(22) Date of filing: **04.10.83**

(54) **Polyolefin compositions.**

(43) Date of publication of application:
**10.04.85 Bulletin 85/15**

(45) Publication of the grant of the patent:
**08.06.88 Bulletin 88/23**

(84) Designated Contracting States:
**AT BE CH DE FR GB IT LI LU NL SE**

(56) References cited:
**DE-A-1 937 384**
**GB-A-1 001 945**
**GB-A-1 422 454**
**US-A-2 906 727**
**US-A-4 431 497**

(73) Proprietor: **MILLIKEN RESEARCH CORPORATION**
**Iron Ore Road**
**Spartanburg South Carolina 29304 (US)**

(72) Inventor: **Rekers, John William**
**530 Poplar Street**
**Spartanburg South Carolina 29302 (US)**

(74) Representative: **Perry, Robert Edward et al**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 136 381 B1

**Description**

The present invention relates to compositions of olefinic polymers intended for use where there is high energy radiation. More particularly, the present invention relates to olefinic polymer compositions which are stable to sterilising dosages of high energy radiation such as gamma radiation.

Olefinic polymers, such as polyethylene and polypropylene, have a wide variety of known applications. US—A—3940325 discloses that olefinic polymers are useful in the manufacture of shaped articles for medical uses and for food packaging, where the articles must undergo sterilisation or be disinfected. It has also been reported that sterilisation of such shaped articles may advantageously be accomplished by irradiating the article with high energy radiation such as gamma radiation.

Notwithstanding the significant known advantages of sterilisation by means of high energy radiation, several disadvantages are known to be associated with such sterilisation techniques. First, when treated with radiation energy in an amount sufficient to achieve the desired sterilisation, such polyolefin compositions may become discoloured. US—A—3537967 suggest that this discolouration may occur as the result of, for example, the use of certain additives in the polymer, and the presence of high amounts of catalytic residues such as titanium and chlorine. Simple removal of the additives from the olefinic polymer composition has not been found to be a satisfactory solution to the problem because, as disclosed in US—A—3940325, the physical properties of shaped articles made from polymers without the standard additives after irradiation with, for instance, X-rays, may be disadvantageously degraded.

Some of the most common additives found in polyolefin polymer compositions to be made into shaped articles, especially where increased melt temperatures or higher melt index polymers are required, are the socalled primary anti-oxidants employed to retard radical chain oxidation. The most common primary anti-oxidants are phenolic in nature, e.g. Goodrite® 3114 and 3125 which are available from B. F. Goodrich Chemical Company. When used at effective concentrations to provide both processing and radiation stability, these compounds have been found to cause the shaped article, which has been irradiated with a sterilising dose, to be unacceptably discoloured.

According to the present invention, a composition comprises an olefinic polymer selected from homopolymers and copolymers of $C_{2-10}$ aliphatic, ethylenically-unsaturated monomers, and from 100 to 10,000 ppm of a compound which is benzohydrol or a benzohydrol derivative and which has the formula

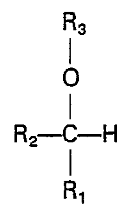

wherein $R_1$ and $R_2$ are the same or different $C_{6-26}$ aromatic groups and $R_3$ is hydrogen, $C_{1-20}$ alkyl or $C_{2-20}$ acyl. Any one of $R_1$, $R_2$ and $R_3$ may carry one or more substituents within the scope defined above (but not necessarily the same as) $R_1R_2C(OR_3)$—, e.g. $R_1R_2COH$— or, often, $R_1CHOH$—. Usually there will be no more than 3, and often no more than 1, such substituents. $R_3$ may be aryl-substituted.

Examples of $R_1$ and $R_2$ include phenyl and substituted phenyl groups. Substituents may be present in any, ortho, meta and/or para, position of the phenyl group and may include one or more alkyl groups of the formula $C_nH_{2n+1}$ wherein n is from 1 to 20, e.g. methyl, ethyl, isopropyl, tert-butyl, nonyl, dodecyl, or eicosyl groups. The same groups illustrate $R_3$ as alkyl; examples of $R_3$ as acyl are acetyl, propionyl, lauryl and stearyl groups.

The benzohydrol compounds (including benzohydrol itself) which are used according to the present invention may be broadly described as stabilisers. These stabilisers are used in an amount of from 100 to 10,000, preferably 500 to 5,000, parts per million (ppm) based on the weight of the total composition. Examples of preferred stabilisers that may be used are benzohydrol and the benzohydrol derivatives of Examples 1 to 5 (below).

Compositions of the present invention may include the benzohydrol compound as the sole stabiliser, or the benzohydrol compound as a stabiliser together with one or more so-called secondary anti-oxidants or synergists. Many secondary anti-oxidants are known for use in association with phenolic-type primary stabilisers; they may function by converting harmful peroxide compounds present in the polymeric composition to non-harmful, non-radical products. Examples of such secondary antioxidants include dilauryl thiodipropionate, distearyl thiodipropionate, trisnonylphenyl phosphite and dilauryl phosphite, and also Weston 618® and Weston 619 which are phosphorus-containing anti-oxidants available from Borg Warner. Such secondary stabilisers may be used in an amount of from 100 to 10,000, preferably 500 to 3,000 ppm.

Compositions of the invention may contain one or more known, phenolic-based, primary stabilisers, such as hindered phenolic-type compounds, in addition to the benzohydrol compound. This is particularly significant, since it may not be possible, in practice, to process polypropylene without a phenolic primary anti-oxidant, the presence of which may lead to yellowing on irradiation. The present invention may

provide a remedy for this phenomenon. In such instances, even stabilisers which have been observed to cause discolouration in the polymer composition when subjected to radiation sterilisation may not cause such discolouration when the benzohydrol compound is also present in the composition. When present, such primary stabilisers may be provided in the composition in an amount of from 100 to 3,000, preferably 500 to 2,000, ppm.

Olefinic polymers which may be present in compositions of the present invention include a wide range of olefinic homopolymers and copolymers of ethylene, propylene, butylene and higher homologues containing up to 10 carbon atoms. Typically, such polymers may have a molecular weight of from 10,000 to 500,000, preferably 30,000 to 300,000. The preferred polymers are homopolymers of propylene and random or block copolymers of propylene with other mono-α-olefins such as ethylene, 1-butene and higher homologues containing up to 10 carbon atoms. If desired, such propylene polymers may be blended with other polymers such as polyethylene. Generally, the proportion of polymerised propylene in the total resin phase of the composition is at least 60% by weight.

In general, the radiation treatment is applied to polymers of propylene intended for medical purposes. A composition of the invention may also be used for other purposes where such radiation treatment is necessary or desirable, e.g. meat packaging and preserving food in retail packages.

The high energy radiation treatment may be, for example, from a cobalt-60 source, or high energy X-rays or high energy electrons (β-radiation). In general, radiation dosages can be applied at up to 5 megarads. For sterilisation purposes, a shaped article is usually treated by applying 2.5 megarads under gamma radiation.

The following Examples 1 to 5 illustrate benzohydrol compounds used in the invention. Examples 6 and 7 illustrate the invention, in comparison with examples of the prior art.

Example 1

4,4′-Dimethylbenzohydrol

To the Grignard reagent prepared from 47.2 g p-bromotoluene and 7.2 g magnesium in 200 ml of ether were added dropwise 30 g p-tolualdehyde in 50 ml ether, with cooling. When the addition was complete, the reaction was refluxed for 15 minutes, cooled in ice, and 40 ml of saturated $NH_4Cl$ solution were added dropwise. The reaction mixture was filtered, and the ether was removed under reduced pressure to give a yellow oil which crystallised on standing. Recrystallisation from petroleum ether gave 26.1 g of nearly colourless title product, m.p. 70—71°C (67—68°C).

Example 2

4,4′-Di-*t*-butylbenzohydrol

From 16.7 g p-bromo-t-butylbenzene, 3.2 g magnesium, and 17.5 g p-t-butylbenzaldehyde, following the procedure of Example 1, and after recrystallisation from aqueous ethanol, 10.8 g of the colourless title compound, m.p. 91—94°C, were obtained.

Example 3

1,4-Dibenzylolbenzene

To a stirred slurry of 56 g aluminium chloride in 150 ml benzene, under reflux, were added dropwise 40 g terephthaloyl chloride dissolved in 250 ml benzene. When the addition was complete, the reaction was refluxed for 15 minutes, cooled, and 250 ml water were added slowly. The benzene layer was separated, and the aqueous layer was extracted with methylene chloride (2×300 ml). The combined organic layers were washed with 1*M* NaOH solution (2×400 ml) and water (400 ml), dried ($MgSO_4$), filtered, and the solvents were removed under reduced pressure to a nearly colourless solid. Recrystallisation from 95% ethanol gave 46.8 g of colourless product, m.p. 160—162°C. Reduction of 20 g of this material with 1.8 g sodium borohydride in 250 ml ethanol gave 14.2 g colourless 1,4-dibenzylolbenzene m.p. 142—145°C.

Example 4

1,4-Di(4′-t-butylbenzylol)benzene

From 28 g aluminium chloride, 275 ml t-butylbenzene, and 20 g terephthalyl chloride, following the procedure of Example 3, 11.8 g of colourless material, m.p. 159—161°C, was obtained. Reduction of 20 g of this material with 1.8 g of sodium borohydride in 250 ml of ethanol gave 16.1 g of colourless title compound, m.p. 156—158°C.

Example 5

Dibenzohydryl ether

A mixture of 37 g chlorodiphenylmethane and 100 ml water was refluxed under nitrogen for 16 hours. After cooling, the water was decanted and 40 ml of ethanol were added to induce crystallisation. The yellow solid was recrystallised 3 times from ethanol to give 21.9 g of title compound, m.p. 108—110°C.

Example 6

0.3% by weight of an additive (given in Table I) was blended into polypropylene powder (Hercules Profax 6301). Pellets were extruded and then injection-moulded into plaques 1.4 mm thick and standard

tensile bars. The moulded sample was irradiated to a 10 Mrad dose with a cobalt-60 γ-ray source. The yellowness index was determined on a Hunter Colorimeter (ASTM D 1925). Percentage elongation at break was used as a measure of embrittlement and was recorded on an Instron testing machine using a strain rate of 127 mm/min (5 in/min, ASTM D 638). This data is given for a control (no additive) and for various additives, in Table I.

TABLE I

| Additive | Yellowness index | Elongation at break (%) |
|---|---|---|
| none | 6.30 | 8.5 |
| benzohydrol | 3.16 | 21.9 |
| dibenzohydryl ether | 3.69 | 18.8 |
| 4,4'-dimethylbenzohydrol | 5.68 | 33.6 |
| 4,4'-di-t-butylbenzohydrol | 3.81 | 22.5 |
| 1,4-dibenzylolbenzene | 3.48 | 23.8 |
| 1,4-di(4'-t-butylbenzylol)benzene | 6.35 | 28.3 |
| Goodrite 3114 | 24.00 | 26.4 |
| Goodrite 3125 | 13.79 | 32.0 |

Example 7

Four samples containing benzohydrol with and without commercial anti-oxidants, and one with a known anti-oxidant alone, were prepared, irradiated, and tested as in Example 6. The test results are shown in Table II.

TABLE II

| Sample | Additive (% concentration) | | Yellowness index | Elongation at break (%) |
|---|---|---|---|---|
| 1 | benzohydrol | (0.3%) | 3.16 | 21.9 |
| 2 | benzohydrol<br>dilauryl thiodipropionate | (0.3%)<br>(0.1%) | 4.97 | 32.2 |
| 3 | benzohydrol<br>Weston 619 | (0.3%)<br>(0.1%) | 3.90 | 31.5 |
| 4 | Goodrite 3114 | (0.1%) | 11.55 | 19.6 |
| 5 | Goodrite 3114<br>benzohydrol | (0.1%)<br>(0.3%) | 8.66 | 26.2 |

**Claims**

1. A polymer composition which comprises an olefinic polymer selected from homopolymers and copolymers of $C_{2-10}$ aliphatic, ethylenically-unsaturated monomers, and from 100 to 10,000 ppm of a compound which is benzohydrol or a benzohydrol derivative and which has the formula

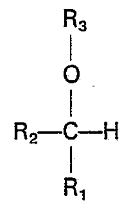

wherein $R_1$ and $R_2$ are the same or different $C_{6-26}$ aromatic groups and $R_3$ is hydrogen, $C_{1-20}$ alkyl or $C_{2-20}$ acyl.

2. A composition according to claim 1, wherein the benzohydrol or benzohydrol derivative is benzohydrol, 4,4'-dimethylbenzohydrol, $Ph_2CHOCHPh_2$, 4,4'-di-t-butylbenzohydrol, 1,4-dibenzylolbenzene or 1,4-di(4'-t-butylbenzylol)benzene.

3. A composition according to claim 1 or claim 2, wherein the benzohydrol or benzohydrol derivative is a primary stabiliser, and which additionally comprises a secondary stabiliser in an amount of from 100 to 10,000 ppm.

4. A composition according to any preceding claim, wherein the benzohydrol or benzohydrol derivative is a primary stabiliser, and which additionally comprises at least one other phenolic-type primary stabiliser in an amount of from 100 to 3,000 ppm.

5. A composition according to any preceding claim, wherein the olefinic polymer is a polypropylene homopolymer or a random or block copolymer of propylene with at least one other mono-α-olefin containing up to 10 carbon atoms.

**Patentansprüche**

1. Polymerzusammensetzung, welche ein Olefinpolymer, ausgewählt unter Homopolymeren und Copolymeren von $C_{2-10}$ aliphatischen, ethylenisch ungesättigten Monomeren, und von 100 bis 10.000 Teile pro Million (ppm) einer Verbindung umfaßt, welche Benzhydrol oder ein Benzhydrolderivat ist und die Formel

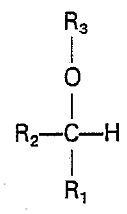

aufweist, worin $R_1$ und $R_2$ gleiche oder verschiedene $C_{6-26}$ aromatische Gruppen bedeuten und $R_3$ Wasserstoff, $C_{1-20}$ Alkyl oder $C_{2-20}$ Acyl bedeutet.

2. Zusammensetzung nach Anspruch 1, worin das Benzhydrol oder Benzhydrolderivat Benzhydrol, 4,4'-Dimethylbenzhydrol, $Ph_2CHOCHPh_2$, 4,4'-Di-tert.butyl-benzhydrol, 1,4-Dibenzylolbenzol oder 1,4-Di(4'-tert.butylbenzylol)benzol ist.

3. Zusammensetzung nach Anspruch 1 oder 2, worin das Benzhydrol oder Benzhydrolderivat ein Primärstabilisator ist und worin die Zusammensetzung zusätzlich einen Sekundärstabilisator in eine Menge von 100 bis 10.000 ppm umfaßt.

4. Zusammensetzung nach einem vorstehenden Anspruch, worin das Benzhydrol oder Benzhydrolderivat ein Primärstabilisator ist und worin die Zusammensetzung zusätzlich wenigstens einen anderen Primärstabilisator vom Phenol-Typus in einer Menge von 100 bis 3.000 ppm umfaßt.

5. Zusammensetzung nach einem vorstehenden Anspruch, worin das Olefinpolymer ein Polypropylen-Homopolymer oder ein statistisches oder Blockcopolymer von propylen mit wenigstens einem anderen, bis zu 10 Kohlenstoffatome enthaltenden Mono-α-olefin ist.

**Revendications**

1. Une composition de polymère qui comprend un polymère oléfinique choisi parmi les homopolymères et copolymères de monomères éthyléniques aliphatiques en $C_2$—$C_{10}$, et de 100 à 10 000 ppm d'un composé qui est le benzhydrol ou un dérivé du benzhydrol et qui répond à la formule

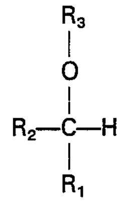

dans laquelle $R_1$ et $R_2$ sont identiques ou différents et représentent des groupes aromatiques en $C_6$—$C_{26}$ et $R_3$ est l'hydrogène ou un groupe alkyle en $C_1$—$C_{20}$ ou acyle en $C_2$—$C_{20}$.

2. Une composition selon la revendication 1, dans laquelle le benzhydrol ou dérivé de benzhydrol est le benzhydrol, le 4,4'-diméthylbenzhydrol, $Ph_2CHOCHPh_2$, le 4,4'-di-t-butylbenzhydrol, le 1,4-dibenzylolbenzène ou le 1,4-di(4'-t-butylbenzylol)benzène.

3. Une composition selon la revendication 1 ou 2, dans laquelle le benzhydrol ou dérivé de benzhydrol est un stabilisant primaire et qui comprend en outre un stabilisant secondaire en quantité de 100 à 10 000 ppm.

4. Une composition selon l'une quelconque des revendications précédentes, dans laquelle le benzhydrol ou dérivé de benzhydrol est un stabilisant primaire et qui comprend en outre au moins un autre stabilisant primaire du type phénolique en quantité de 100 à 3 000 ppm.

5. Une composition selon l'une quelconque des revendications précédentes, dans laquelle le polymère oléfinique est un homopolymère de polypropylène ou un copolymère statistique ou séquencé de propylène avec au moins une autre mono-α-oléfine jusqu'en $C_{10}$.